# EUROPEAN PATENT SPECIFICATION

(11) **EP 2 334 325 B1**
(45) Date of publication and mention of the grant of the patent: **15.03.2017**
(21) Application number: 08816736.6
(22) Date of filing: 14.10.2008
(51) Int. Cl.: A61K 39/00, A61K 39/38, A61K 39/12, A61K 39/13

(54) **PROCESS FOR TREATMENT OF RHEUMATOID ARTHRITIS, TREMORS/PARKINSON'S DISEASE, MULTIPLE SCLEROSIS AND NON-VIRAL BASED CANCERS**
VERFAHREN ZUR BEHANDLUNG VON RHEUMATOIDER ARTHRITIS, TREMOR/PARKINSON-KRANKHEIT, MULTIPLER SKLEROSE UND NICHT-VIRALEN KREBSARTEN
MÉTHODE DE TRAITEMENT DE LA POLYARTHRITE RHUMATOÏDE, DES TREMBLEMENTS/LA MALADIE DE PARKINSON, DE LA SCLÉROSE EN PLAQUES ET DE CANCERS D'ORIGINE NON VIRALE

(30) Priority: 26.09.2008 WO PCT/US2008/011233
(43) Date of publication of application: 22.06.2011
(73) Proprietor: Honor C.W. M.D., LLC, Avalon, CA 90704 (US)
(72) Inventor: NELSON, George, Chatsworth, CA 91313 (US)
(74) Representative: Patentgruppen A/S
(86) International application number: PCT/US2008/011775
(87) International publication number: WO 2010/036230

(56) References cited:
- US-A1- 2006 045 884
- US-A1- 2007 071 722
- US-A1- 2007 253 973
- US-A1- 2008 146 488
- US-A1- 2008 176 946
- US-B1- 6 759 241
- DIEZ-DOMINGO JAVIER ET AL: "Immunogenicity and reactogenicity of a combined adsorbed tetanus toxoid, low dose diphtheria toxoid, five component acellular pertussis and inactivated polio vaccine in six-year-old children", PEDIATRIC INFECTIOUS DISEASE JOURNAL, LIPPINCOTT WILLIAMS & WILKINS, US, vol. 34, no. 3, 1 March 2005 (2005-03-01), pages 219-224, XP009135687, ISSN: 0891-3668, DOI: 10.1097/01.INF.0000154339.72774.B0
- SHOENFELD Y ET AL: "Vaccination and Autoimmunity-'vaccinosis': A Dangerous Liaison?", JOURNAL OF AUTOIMMUNITY, vol. 14, no. 1, 1 February 2000 (2000-02-01), pages 1-10, XP055193660, ISSN: 0896-8411, DOI: 10.1006/jaut.1999.0346

## Description

### FIELD

The present invention relates generally to compositions for use in the treatment of autoimmune disorders, and specifically, to the treatment of demyelinating diseases such as rheumatoid arthritis, Tremors/Parkinson's Disease and multiple sclerosis. The present invention also relates to compositions for use in the treatment of non-viral based cancers.

### BACKGROUND

Rheumatoid arthritis ("RA") is an autoimmune disease that is typically manifest by inflammation of the synovial joints. The development of RA progresses chronically, alternating between remission and relapse. Damage and deformation of joints can occur rapidly, particularly if the disease is untreated. As the disease progresses, RA can cause joint destruction, functional disability and premature mortality. RA can also include systemic inflammatory disease affecting multiple organs. RA patients often suffer physically and mentally from heavy pain all their lives. The cause of RA is presently unknown.

As an autoimmune disease, RA is characterized by a defect in the body's ability to distinguish foreign molecules from its own. The immune system attacks the synovial membrane, causing inflammation due to the infiltration of the membrane with T cells, plasma cells and macrophages. Formation of granulation tissue at the edges of the synovial lining is marked by extensive angiogenesis and enzyme production. These effects in turn cause progressive, erosive disintegration of adjacent cartilage and bone. In conjunction with the inflammation of the membranes, patients suffering from RA can also exhibit nerve abnormalities that primarily seem to involve segmental destruction of the myelin sheath.

Early stage prior art treatments typically attempt to ameliorate the pain symptoms through administration of non-steroidal anti-inflammatory drugs (NSAID). However, these treatments do little or nothing to affect the progression of RA.

Once a definitive RA diagnosis is made, conventional treatments include the use of steriods in conjunction with physical therapy and, if joint damage occurs, surgery. Again, these treatments have significant drawbacks and do not address the underlying causes of RA. For example, steroid therapy is associated with a number of well-known adverse side effects.

Specific compounds known as disease modifying anti-rheumatic drugs (DMARD) have been developed in an attempt to directly target the processes associated with RA. These DMARDs are typically administered in conjunction with NSAIDs. Examples of such compounds include Remicade®, methotrexate, and Humira®, which are all immunomodulators designed to inhibit the function of the body's immune system. While such treatments can slow the attack of RA, they undermine the ability of the immune system to respond normally to infections and leave the patient vulnerable to other diseases. Furthermore, they do not address the underlying causes of RA. Moreover, there are potentially severe side effects from using these immomodulators and there are restrictions placed on users to avoid exercise, alcohol and to be concerned about drug interferences.

As no cure for RA exists, there exists a need for treatments that alleviate the pain and inflammation associated with RA without the drawbacks inherent in prior art strategies. Similarly, there is a need for treatments that mitigate the joint damage associated with RA. One object of the current invention is to provide such treatments while minimizing the negative effects on a patient's immune system.

In addition to RA, there are a number of other progressive or degenerative diseases, such as Crohn's disease, multiple sclerosis ("MS"), Tremors/Parkinson's Disease, Alzheimer's disease, amyotrophic lateral sclerosis ("ALS"), Guillain-Barre syndrome, atherosclerosis, schizophrenia, Parkinsons's disease, senile dementia and others, associated with nerve damage. Although distinct, these diseases share common elements. Specifically, the precise origin or cause of these diseases remains unknown, yet they all exhibit damage to the nerves in the form of demyelination. As with RA, there is currently no cure for these diseases and prior art treatments have focussed on modulating the patient's immune system. For example, Copaxone® is administered to patients suffering from MS in order to suppress immune response. Naturally, a significant side effect of such treatments is the potential for the patient to have a compromised immune system.

Accordingly, there exists a need for treatments for MS, Alzheimer's disease, Parkinson's disease and the like that minimize the drawbacks associated with the prior art. Similarly, there is a need for a treatment for such diseases that helps prevent demyelination.

In certain cancers, there may be a latent viral infection that remains quiescent until some signal triggers a release from latency. Once triggered, the tumorous cell begins to replicate. The identification or disease etiology is difficult to assign because in some infections, the DNA of the causation virus is integrated into the genome of the host cell and is transmitted vertically. It therefore behaves as a genetic attribute. In other circumstances, the causative microbe triggers the cancer-disease process and then disappears from the body and is no longer detectable. What is needed, therefore is a vaccine that prevents single strand linear viruses from triggering the release of cancer from latency. It is these types of cancers, such as e.g., prostrate, liver, pancreatic, and lung cancer, that are referred to as the non-viral based cancers. Non-viral based cancers are to be contrasted with viral cancers whose etiology has been directly traced to viral causes. At present, only two viruses, human T-cell lymphotropic virus and human papillomavirus, are considered to be human tumor viruses. However, several other candidate viruses are implicated by epidemiological correlation, by serologic relationship or by recovery of virus from tumor cells.

Diez-Domingo et al., (The Pediatric Infectious Disease Journal, March 2005; 24(3): 219-224) discloses immunogenicity and reactogenicity of a combined absorbed tetanus toxoid, low dose diphtheria toxoid, five component acellular pertussis and inactivated polio vaccine in six-year-old children.

US 6759241 B1 (Hone et al.) discloses an adjuvant comprising a lipopolysaccharide antagonist.

### SUMMARY OF THE INVENTION

The present invention provides a composition comprising an immunity-provoking agent and a bacterial antigen activator, wherein the immunity-provoking agent comprises an inactivated polio vaccine and wherein the bacterial antigen activator comprises tetanus toxoid and typhim VI.

Further, the present invention provides the composition for use in medicine.

The present invention also provides a composition comprising an immunity-provoking agent and a bacterial antigen activator for use in treating pain and inflammation in a patient, wherein the immunity-provoking agent comprises an inactivated polio vaccine and wherein the bacterial antigen activator comprises tetanus toxoid and typhim VI.

Still further, the present invention provides a composition comprising an immunity-provoking agent and a bacterial antigen activator for use in treating non-viral based cancers in a patient, wherein the immunity-provoking agent comprises an inactivated polio vaccine and wherein the bacterial antigen activator is selected from the group comprising tetanus toxoid and typhim VI.

In addition, the present invention provides the use of an immunity-provoking agent and a bacterial antigen activator for the manufacture of a medicament for treatment of a patient suffering from pain and inflammation or a non-viral based cancer, wherein the immunity-provoking agent comprises an inactivated polio vaccine and wherein the bacterial antigen activator comprises tetanus toxoid and typhim VI.

The present description is directed to compositions useful in treating symptoms of diseases associated with demyelination of the nerves, such as RA, MS, Tremors/Parkinson's Disease and non-viral based cancers. The composition includes an immunity-provoking agent and a bacterial antigen activator. Preferably, the immunity-provoking agent is a vaccine for a single-stranded RNA virus and more preferably, the immunity-provoking agent is an inactivated polio vaccine. Also preferably, the bacterial antigen activator is either or both tetanus toxoid and typhim VI.

Preferably, the composition comprises 5 parts of the inactivated polio vaccine to 1 part of the tetanus toxoid and 1 part of the typhim VI. Alternatively, the composition comprises 5 parts of the inactivated polio vaccine to 2 parts of either tetanus toxoid or typhim VI.

Also preferably, the composition is formulated for subcutaneous injection.

Another aspect of the description is directed to a method for treating pain and inflammation in a patient comprising the steps of preparing a composition of an immunity-provoking agent and a bacterial antigen activator; and administering the composition to the patient. Preferably, the step of administering the composition comprises administering the composition subcutaneously. More preferably, the step of administering the composition comprises administering approximately 70 cc of the composition.

The method includes treating a patient suffering from a demyelinating disease. Examples of such diseases include rheumatoid arthritis, multiple sclerosis, Alzheimer's disease, ALS, Guillain-Barre syndrome, atherosclerosis, schizophrenia, Tremors/Parkinsons's disease, and senile dementia.

The method includes treating a patient suffering from a non-viral based cancer disease. Examples of such cancer diseases include prostate cancers. The treatment of these disease conditions according to the compositions and methods of the invention eliminate the restrictions placed on the user's of prior art immunomodulators and the potentially severe side effects of these compounds.

### DETAILED DESCRIPTION

The present description provides a process for treating diseases associated with demyelination of the nerves, such as RA, MS, Alzheimer's disease, ALS, Guillain-Barre syndrome, atherosclerosis, schizophrenia, Tremors/Parkinsons's disease, and senile dementia, and for treating non-viral based cancers. By administering measured doses of an immunity-provoking agent and a bacterial antigen activator, patients suffering from these diseases and cancers have realized beneficial results. In connection with the non- viral based cancer diseases, the vaccination should be used, as appropriate, along with surgery, radiation and chemotherapy. However, as a vaccine, the present composition has the ability to combat the genesis of the non-viral cancer disease.

As discussed above, there exist a significant class of diseases for which the causative agents are poorly understood, but share a common symptom of nerve damage due to demyelination.

Myelin is the protective sheath around axons in the nervous system, also known as "white matter." Myelin insulates the nerve and facilitates the conduction of the electrical potential associated with a neuronal signal. The myelin sheath is composed of glycolipids and proteins deposited around the axon by glial cells. Myelination of the nerves is an ongoing process that occurs during development and throughout childhood.

Demyelination can occur when the patient's immune system attacks the sheath, removing portions of the myelin from the axon. The physiological response to this damage causes the formation of gliotic plaques that interfere with conduction of the nerve impulses.

Without being limited to a particular theory, it is proposed that viral infection causes the patient's myelin to become targeted by the immune system. In response to the infection, the immune system produces antibodies to antigens associated with the infectious agent. However, when these antibodies are insufficiently specific and also recognize normal host antigens, such as components of the myelin sheath, a destructive, autoimmune response can result. Specifically, a dormant childhood infection could form the basis for a subsequent immune response that leads to one of the noted neurodegenerative diseases. Triggers for such a response could be severe physical/psychological trauma or it could be exposure to a suitable antigen or even the natural completion of the myelination process during the transition into adulthood.

In a related modality, a dormant childhood infection can also form the basis for triggering the replication of cancerous cells that have been in a latent state.

Accordingly, treatment with a suitable vaccine should counter this effect and compositions of the description include an immunity-provoking agent.

Suitable immunity-provoking agents are preparations, such as vaccines, having the ability to confer a degree of immunity to a patient for a demyelinating disease. Preferably, the disease is also known to have the ability to penetrate the central nervous system ("CNS") of the patient.

In one aspect, the immunity-provoking agent comprises a polio vaccine. Poliomyelitis is a disease characterized by degradation of the myelin sheath, often leading to paralysis. The polio virus is a human enterovirus and member of the family of Picornaviridae composed of a single-stranded positive-sense RNA genome and protein capsid. Although a majority of polio infections are asymptomatic, in a small percentage of cases the virus does invade the patient's CNS5 leading to the nerve damage that is the primary symptom of the disease. More preferably, the immunity-provoking agent comprises inactivated polio vaccine ("IPV"), such as trivalent IPV.

Other suitable uses for this vaccine with the single stranded RNA-based viruses that may be used in the practice of the methods described herein include vaccines for rubella, mumps, measles, Rhinovirus virus, hepatitis A virus, Hepatitis C virus, Yellow Fever Virus, Dengue Virus and West Nile Virus.

It has been found that the compositions also require a bacterial antigen activator in conjunction with the immunity-provoking agent. Suitable bacterial antigen activators include gram-negative bacteria vaccines and gram-positive bacteria vaccines. Specific bacterial antigen activators found to be useful in the practice of the methods described herein include tetanus toxoid and typhoid vaccine.

*Clostridium tetani* is a gram-positive, obligate anaerobic bacterium that produces the neurotoxin tetanospasmin. Tetanus toxoid is a modified form of tetanospasmin shown to stimulate the production of suitable antibodies and confer an immunity to tetanus. *Salmonella enterica serovar typhi* is a gram-negative, flagellated, rod-shaped bacterium and is the disease agent in typhoid fever. Typhoid vaccines are prepared from antigens particular to the bacterium. For example, the typhim VI vaccine is prepared from a cell surface polysaccharide of *S. typhi.*

The use of Diphtheria Toxoid to develop another vaccine to a single-strand virus is also intended to be within the scope of the description.

Accordingly, a presently preferred aspect a composition for subcutaneous injection comprising IPV, typhim VI and tetanus toxoid. More specifically, the composition preferably comprises 1 part tetanus toxoid, 1 part typhim VI, and 5 parts IPV. Alternatively, the composition comprises 2 parts tetanus toxoid and 5 parts IPV. In another alternative, the composition comprises 2 parts typhim VI and 5 parts IPV. The above ratios are all based on concentrations of IPV at (80 D antigen units Type 1)/mL, (16 D antigen units Type 2)/mL, and (64 D antigen units Type 3)/mL, tetanus toxoid at 10 Lf (flocculation units)/mL and 2 units antitoxin/mL, and typhim VI at 50 mg/mL

The frequency and size of the vaccine dosage can be increased or decreased according to the patient's physical stature, and the general nature of the patient's health. However, preferably, the dosage remains at 70 cc per treatment.

For treatment in a patient suffering from pain and inflammation, the method comprises the steps of preparing a composition of immunity-provoking agent and bacterial antigen activator and administering the composition to the patient.

Preferably, the methods are directed to treatment of symptoms associated with RA, MS, Alzheimer's disease, ALS, Guillain-Barre syndrome, atherosclerosis, schizophrenia, Parkinsons's disease, senile dementia, and other diseases characterized by demyelization, and furthermore to the treatment of non-viral based cancers.

As noted above, the composition of the method preferably comprises 1 part tetanus toxoid, 1 part typhim VI, and 5 parts IPV, or 2 parts tetanus toxoid and 5 parts IPV, or 2 parts typhim VI and 5 parts I PV.

Also preferably, the step of administering the composition comprises subcutaneously injecting 70 cc of the composition.

Case Studies for Rheumatoid Arthritis (RA), Multiple Scleroses (MS), and Tremor's/Parkinson's (P) and Prostrate Cancer (PC).

### Rheumatoid Arthritis (RA)

1. RA is a 61 year old male who has suffered from rheumatoid arthritis in his hands, fingers and back for the last 10 years. He started the medication 5 years ago and within one hour after taking the medication, the pain in his hands, fingers and back disappeared and by the second medication he continued to have no pain and no limitations of movement. He is basically symptom free of his rheumatoid arthritis and has continued taking the medication on a weekly basis. Absolutely no side effects.
2. RA is a 63 year old female who gave up golf as a result of rheumatoid arthritis. She has it in her hands, as well as her wrists and believes in her back for 10 years. She started the medication 2 years ago and within 45 minutes after the medication was administered, she was basically pain free, and had full and complete movement of both her wrists, hands and noticed no back pain whatsoever. She takes the medication once every 5 days and continues to remain pain free. Absolutely no side effects.
3. RA is a 82 year old man who had severe rheumatoid arthritis for 20 years. For the last 20 years both of his hands were clenched in a fist position and he suffered with severe pain in his hands. He received his first medication 3 years ago. After 45 minutes taking the medication he was crying for joy because this was the first time in 20 years he was without pain and an hour and a half after medication he was able to open his hands one inch. As his treatment continued every 5 days he regained full use of his hands with no pain and absolutely no side effects.

### Multiple Scleroses (MS)

1. MS is a 62 year old female patient who has advanced MS. For eight years she suffered with severe pain in the right leg and was confined to a wheelchair, had incontinence, dysentery and multiple brain sheers (her doctor states that the last time she had seen a patient with this many brain sheers, it was a corpse). She started her medication 2 ½ years ago. Her first medication reduced her pain by 50% and the 2^{nd} medication 2 days later, within 45 minutes had no pain at all. The 3^{rd} medication 4 days later she was still pain free and was able to stand and use a walker to help her get around. The 4^{th} medication just 4 days later, she still showed no signs of pain, incontinence or dysentery and had no side effects. She began taking the medication every 5 days to maintain a healthy pain free life still with no dysentery and absolutely no side effects.

### Tremor's/Parkinson's Disease (P)

1. P is a 64 year old man who noticed an occasional slight tremor in his left hand one year ago. He thought it was nerves. As time went on, the tremors were more frequent. He consulted with his doctor and was told it was it could be nerves or the beginning of Parkinson's Disease but there was no way to tell without an autopsy (not an option.) He tried compound vitamins, no help. After his first shot of the medication, the tremors stopped within 45 minutes, with no side effects. One week later, the left hand started some movement, I gave him another shot and the movement/tremors stopped. He has taken weekly shots since, and there have been no tremors and no side effects.

### Prostate Cancer

Twelve years ago P had a PSA score of 68 and a Gleason score of 7. A radical prostate ectomy was performed, and P was given a prognosis of one to two years additional life. After P began administering the vaccination of the present invention, P's PSA score was -0.03 and has remained that way for twelve years.

## Claims

1. A composition comprising an immunity-provoking agent and a bacterial antigen activator, wherein the immunity-provoking agent comprises an inactivated polio vaccine and wherein the bacterial antigen activator comprises tetanus toxoid and typhim VI.

2. The composition of claim 1, comprising 5 parts of the inactivated polio vaccine to 2 parts of the bacterial antigen activator.

3. The composition of claim 1, comprising 5 parts of the inactivated polio vaccine to 1 part of the tetanus toxoid and 1 part of the typhim VI.

4. The composition of claim 1, wherein the composition is formulated for subcutaneous injection.

5. A composition according to any preceding claim for use in medicine

6. A composition comprising an immunity-provoking agent and a bacterial antigen activator for use in treating pain and inflammation in a patient, wherein the immunity-provoking agent comprises an inactivated polio vaccine and wherein the bacterial antigen activator comprises tetanus toxoid and typhim VI.

7. A composition for use according to claim 6, wherein the patient is suffering from a disease **characterized by** demyelination.

8. A composition for use according to claim 7, wherein the disease is selected from the group consisting of rheumatoid arthritis, multiple sclerosis, Alzheimer's disease, ALS, Guillain-Barre syndrome, atherosclerosis, schizophrenia, Parkinsons's disease, and senile dementia.

9. A composition comprising an immunity-provoking agent and a bacterial antigen activator for use in treating non-viral based cancers in a patient, wherein the immunity-provoking agent comprises an inactivated polio vaccine and wherein the bacterial antigen activator is selected from the group comprising tetanus toxoid and typhim VI.

10. A composition for use according to claim 9 wherein the patient is suffering from a disease **characterized by** cancer cell replication triggered by dormant infections **characterized by** single strand linear virus.

11. A composition for use according to claim 10, wherein the disease is selected from the group consisting of prostate cancer, brain cancer, lung cancer, breast cancer and pancreatic cancer.

12. A composition for use according to any one of claims 6 to 11, wherein the composition comprises 5 parts of the inactivated polio vaccine to 2 parts of the bacterial antigen activator.

13. A composition for use according to any one of claims 6 to 11, wherein the composition comprises 5 parts of the inactivated polio vaccine to 1 part of the tetanus toxoid and 1 part of the typhim VI.

14. A composition for use according to any one of claims 6 to 13 wherein the composition is for subcutaneous administration.

15. Use of an immunity-provoking agent and a bacterial antigen activator for the manufacture of a medicament for treatment of a patient suffering from pain and inflammation or a non-viral based cancer, wherein the immunity-provoking agent comprises an inactivated polio vaccine and wherein the bacterial antigen activator comprises tetanus toxoid and typhim VI.

## Patentansprüche

1. Zusammensetzung, umfassend ein Immunität hervorrufendes Agens und einen bakteriellen Antigen-Aktivator, wobei das Immunität hervorrufende Agens einen inaktivierten Polioimpfstoff umfasst und wobei der bakterielle Antigen-Aktivator Tetanus-Toxoid und Typhim VI umfasst.

2. Zusammensetzung nach Anspruch 1, umfassend 5 Teile des inaktivierten Polioimpfstoffs zu 2 Teilen des bakteriellen Antigen-Aktivators.

3. Zusammensetzung nach Anspruch 1, umfassend 5 Teile des inaktivierten Polioimpfstoffs zu 1 Teil des Tetanus-Toxoid und 1 Teil des Typhim VI.

4. Zusammensetzung nach Anspruch 1, wobei die Zusammensetzung zur subkutanen Injektion formuliert ist.

5. Zusammensetzung gemäß irgendeinem vorhergehenden Anspruch zur Verwendung in der Medizin.

6. Zusammensetzung, umfassend ein Immunität hervorrufendes Agens und einen bakteriellen Antigen-Aktivator, zur Verwendung bei der Behandlung von Schmerzen und Entzündungen bei einem Patienten, wobei das Immunität hervorrufende Agens einen inaktivierten Polioimpfstoff umfasst und wobei der bakterielle Antigen-Aktivator Tetanus-Toxoid und Typhim VI umfasst.

7. Zusammensetzung zur Verwendung gemäß Anspruch 6, wobei der Patient an einer durch Demyelinisierung gekennzeichneten Krankheit leidet.

8. Zusammensetzung zur Verwendung gemäß Anspruch 7, wobei die Krankheit aus der Gruppe, bestehend aus rheumatoider Arthritis, multipler Sklerose, Alzheimer-Krankheit, ALS, Guillain-Barré-Syndrom, Atherosklerose, Schizophrenie, Parkinson-Krankheit und Altersdemenz, ausgewählt ist.

9. Zusammensetzung, umfassend ein Immunität hervorrufendes Agens und einen bakteriellen Antigen-Aktivator, zur Verwendung bei der Behandlung nicht-viraler Krebsarten bei einem Patienten, wobei das Immunität hervorrufende Agens einen inaktivierten Polioimpfstoff umfasst und wobei der bakterielle Antigen-Aktivator aus der Tetanus-Toxoid und Typhim VI umfassenden Gruppe ausgewählt ist.

10. Zusammensetzung zur Verwendung gemäß Anspruch 9, wobei der Patient an einer Krankheit leidet, die durch Krebszellenreplikation gekennzeichnet ist, welche durch latente, durch einzelsträngige lineare Viren gekennzeichnete Infektionen ausgelöst wird.

11. Zusammensetzung zur Verwendung gemäß Anspruch 10, wobei die Krankheit aus der Gruppe, bestehend aus Prostatakrebs, Gehirntumor, Lungenkrebs, Brustkrebs und Bauchspeicheldrüsenkrebs, ausgewählt ist.

12. Zusammensetzung zur Verwendung gemäß einem der Ansprüche 6 bis 11, wobei die Zusammensetzung 5 Teile des inaktivierten Polioimpfstoffs zu 2 Teilen des bakteriellen Antigen-Aktivators umfasst.

13. Zusammensetzung zur Verwendung gemäß einem der Ansprüche 6 bis 11, wobei die Zusammensetzung 5 Teile des inaktivierten Polioimpfstoffs zu 1 Teil des Tetanus-Toxoid und 1 Teil des Typhim VI umfasst.

14. Zusammensetzung zur Verwendung gemäß einem der Ansprüche 6 bis 13, wobei die Zusammensetzung zur subkutanen Verabreichung ist.

15. Verwendung eines Immunität hervorrufenden Agens und eines bakteriellen Antigen-Aktivators zur Herstellung eines Medikaments zur Behandlung eines Patienten, der an Schmerzen und einer Entzündung oder an einer nicht-viralen Krebsart leidet, wobei das Immunität hervorrufende Agens einen inaktivierten Polioimpfstoff umfasst und wobei der bakterielle Antigen-Aktivator Tetanus-Toxoid und Typhim VI umfasst.

## Revendications

1. Composition comprenant un agent provoquant l'immunité et un activateur d'antigène bactérien, où l'agent provoquant l'immunité comprend un vaccin polio inactivé et où l'activateur d'antigène bactérien comprend l'anatoxine tétanique et le typhim VI.

2. Composition de la revendication 1, comprenant 5 parties du vaccin polio inactivé sur 2 parties de l'activateur d'antigène bactérien.

3. Composition de la revendication 1, comprenant 5 parties du vaccin polio inactivé sur 1 partie de l'anatoxine tétanique et 1 partie du typhim VI.

4. Composition de la revendication 1, dans laquelle la composition est formulée pour une injection sous-cutanée.

5. Composition selon l'une des revendications précédentes pour une utilisation en médecine.

6. Composition comprenant un agent provoquant l'immunité et un activateur d'antigène bactérien pour une utilisation dans le traitement d'une douleur et d'une inflammation chez un patient, où l'agent provoquant l'immunité comprend un vaccin polio inactivé et où l'activateur d'antigène bactérien comprend l'anatoxine tétanique et le typhim VI.

7. Composition pour utilisation selon la revendication 6, dans laquelle le patient souffre d'une maladie **caractérisée par** une démyélinisation.

8. Composition pour une utilisation selon la revendication 7, dans laquelle la maladie est choisie dans le groupe constitué de la polyarthrite rhumatoïde, de la sclérose en plaques, de la maladie d'Alzheimer, de SLA, du syndrome de Guillain-Barré, de l'athérosclérose, de la schizophrénie, de la maladie de Parkinson et de la démence sénile.

9. Composition comprenant un agent provoquant l'immunité et un activateur d'antigène bactérien pour une utilisation dans le traitement des cancers d'origine non virale chez un patient, où l'agent provoquant l'immunité comprend un vaccin polio inactivé et où l'activateur d'antigène bactérien est choisi dans le groupe comprenant l'anatoxine tétanique et le typhim VI.

10. Composition pour une utilisation selon la revendication 9 dans laquelle le patient souffre d'une maladie **caractérisée par** la réplication de cellules cancéreuses déclenchée par des infections dormantes **caractérisées par** un virus à simple brin linéaire.

11. Composition pour une utilisation selon la revendication 10, dans laquelle la maladie est choisie dans le groupe constitué du cancer de la prostate, du cancer du cerveau, du cancer du poumon, du cancer du sein et du cancer du pancréas.

12. Composition pour une utilisation selon l'une quelconque des revendications 6 à 11, dans laquelle la composition comprend 5 parties du vaccin polio inactivé sur 2 parties de l'activateur d'antigène bactérien.

13. Composition pour une utilisation selon l'une quelconque des revendications 6 à 11, dans laquelle la composition comprend 5 parties du vaccin polio inactivé sur 1 partie de l'anatoxine tétanique et 1 partie du typhim VI.

14. Composition pour une utilisation selon l'une quelconque des revendications 6 à 13 dans laquelle la composition est destinée à une administration sous-cutanée.

15. Utilisation d'un agent provoquant l'immunité et d'un activateur d'antigène bactérien pour la fabrication d'un médicament pour le traitement d'un patient souffrant d'une douleur et d'une inflammation ou d'un cancer d'origine non virale, où l'agent provoquant l'immunité comprend un vaccin polio inactivé et où l'activateur d'antigène bactérien comprend l'anatoxine tétanique et le typhim VI.
